(19) Europäisches Patentamt
European Patent Office
Office européen des brevets

(11) **EP 4 459 543 A1**

(12) **EUROPEAN PATENT APPLICATION**

(43) Date of publication:
**06.11.2024 Bulletin 2024/45**

(21) Application number: **23170911.4**

(22) Date of filing: **01.05.2023**

(51) International Patent Classification (IPC):
**G06T 5/50** *(2006.01)* **A61B 6/00** *(2024.01)*
**H04N 23/80** *(2023.01)*

(52) Cooperative Patent Classification (CPC):
**G06T 5/50; A61B 6/032; A61B 6/5235;**
A61B 6/481; A61B 6/482; G06T 2207/10081;
G06T 2207/20221; G06T 2207/30048; H04N 23/80

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **WEESE, Rolf Jürgen
  Eindhoven (NL)**
• **WIEMKER, Rafael
  Eindhoven (NL)**

• **HEESE, Harald Sepp
  5656AG Eindhoven (NL)**
• **CHEN, Hongxin
  Eindhoven (NL)**
• **NI, Wei
  Eindhoven (NL)**
• **NICKISCH, Hannes
  Eindhoven (NL)**
• **PETERS, Jochen
  Eindhoven (NL)**

(74) Representative: **Philips Intellectual Property &
Standards
High Tech Campus 52
5656 AG Eindhoven (NL)**

(54) **DEVICE, SYSTEM AND METHOD FOR SYNTHESIZING A MEDICAL IMAGE OF A REGION OF INTEREST OF A SUBJECT**

(57)   The present invention relates to a device, system and method for synthesizing a medical image of a region of interest (ROI) of a subject. One or more anatomical sub-ROIs in at least one of two or more input images of an input image data set are segmented. A set of weights is determined for synthesizing medical images from the two or more input images using the set of weights. A plurality of medical images are synthesized from the two or more input images using different weights of the determined set of weights. A quality measure is determined for different pairs of anatomical sub-ROIs for the plurality of synthesized medical images, the quality measure indicating differences between the anatomical sub-ROIs of a pair. Weights are selected from the determined set of weights for which the sum of the quality measures determined for the different pairs of anatomical sub-ROIs is maximal. An output medical image is synthesized from the two or more input images using the selected weights or one of the one or more synthesized medical images is selected as output medical image based on the selected weights.

FIG.3

**Description**

FIELD OF THE INVENTION

The present invention relates to a device, a system and a method for synthesizing a medical image of a region of interest (ROI) of a subject, e.g. a patient in a hospital.

BACKGROUND OF THE INVENTION

Spectral CT provides several spectral base images (SBIs), from which different kinds of images (spectral results such as a monoenergetic image (e.g. a monoE40KeV image), a iodine map, a Zeff image, etc.) can be generated. The contrast between different anatomical structures varies in the images and a specific combination or kind of image is best suited for diagnosis and analysis. For instance, the left ventricular myocardium and ventricle are well visible in the monoE40KeV image of a late contrast enhancement (LCE) spectral CT angiography (CTA) scan while the LCE regions are best visible in iodine maps.

For a diagnosis it may be desirable to generate a single medical image that optimally shows the relevant anatomical structures for the clinical application, use case or diagnostic task at hand.

SUMMARY OF THE INVENTION

It is an object of the present invention to provide a device, a system and a method for generating a medical image of ROI of a subject that shows desired anatomical structures in the ROI in an improved manner, which may enable a practitioner to make an improved diagnosis.

In a first aspect of the present invention a device for synthesizing a medical image of a ROI of a subject is presented, the device comprising:

- an input configured to obtain an input image data set of the ROI of the subject, the input image data set comprising two or more input images;
- a processing unit configured to

    - segment one or more anatomical sub-ROIs in at least one of the two or more input images;
    - determine a set of weights for synthesizing medical images from the two or more input images using the set of weights;
    - synthesize one or more medical images from the two or more input images using different weights of the determined set of weights;
    - determine a quality measure for different pairs of anatomical sub-ROIs for the one or more synthesized medical images, the quality measure indicating differences between the anatomical sub-ROIs of a pair;
    - select weights from the determined set of weights for which the sum of the quality measures determined for the different pairs of anatomical sub-ROIs is maximal; and
    - synthesize an output medical image from the two or more input images using the selected weights or select one of the one or more synthesized medical images as output medical image based on the selected weights; and

- an output configured to output the synthesized output medical image.

In a further aspect of the present invention an imaging system for synthesizing a medical image of a ROI of a subject is presented, the imaging system comprising:

- an imaging device configured to acquire an input image data set of a region of interest, ROI, of a subject, the input image data set comprising two or more input images;
- a device for synthesizing a medical image of the ROI of the subject based on the acquired input image data set; and
- a display unit configured to display the synthesized medical image.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to synthesize a medical image, in particular a spectral image such as a spectral CT image, for a specific diagnostic task. For that purpose, a number of anatomical sub-regions of interest, i.e. part of a ROI, are segmented interactively or automatically. A measure, also called quality measure herein, characterizing the contrast-to-noise ratio (CNR) or other differences of neighboring structures is computed in dependence of, for instance, a (e.g. linear) combination of input images (e.g. spectral CT base images) and accumulated for the neighboring structures. The overall quality measure (e.g. CNR measure) is then optimized with respect to the weights of the combination to define the desired synthesized output image.

As input a spectral image data set, e.g. a single spectral CT image, of the ROI of the subject may be used, which generally comprises two or more spectral images, e.g. one or more base images, a monoenergetic (e.g. monoE40KeV) image, an iodine map, a Zeff image (effective atomic number image), etc. Spectral images are generally acquired simultaneously (when using dual layer detectors or photon counting detectors) or almost simultaneously (when using dual sources, where there is a very small time shift).Ik heb een

It shall be noted that a number of input images (e.g. for a predefined set of weights) may be generated by an imaging device (e.g. a CT scanner). For instance, not only base images, but a set of further spectral images may be generated already by the imaging device.

It shall further be noted that the disclosed solution may be applied to multi-contrast MR images instead of spectral CT images.

ROIs can be copied from one input image to other input images. An anatomical sub-ROI should at least be segmented in one of the input images, but the input images might be different for different anatomical sub-ROIs. The segmentation of one anatomical sub-ROI may be sufficient since the area inside the sub-ROI and outside of the sub-ROI may be used as two separate sub-ROIs.

According to an embodiment, the processing unit is configured to determine, as quality measure, a contrast-to-noise ratio, CNR, or a scalar quantity characterizing the gray value differences between two anatomical sub-ROIs of a pair of anatomical sub-ROIs. These represent useful examples leading to reasonable results.

According to another embodiment, the processing unit is configured to synthesize the one or more medical images as weighted compositions of the two or more input images using different weights of the determined set of weights. Similarly, the processing unit may be configured to synthesize the output medical image as weighted composition from the two or more input images using the selected weights. A weighted composition is only one of several possible options to generate new input images from base images. An essential aspect is that the best weights for the application at hand are determined. In a rather simple embodiment one or more monoenergetic images are generated and a grid search is performed, e.g. to generate monoenergetic images for 40, 50, 60, 70, ...,120 kVP (where the parameter is energy) and to select the image with the best contrast for the marked sub-ROIs.

The processing unit may further be configured to determine the set of weights by use of a grid search or from a predetermined set of weights or by use of an optimization of a previously used set of weights. Optimization basically tests an "objective" for a number of parameters and selects the parameters for which the objective is maximal or minimal. The parameters may be predefined (i.e., they do not depend on values of the objective evaluated for some parameters; grid search is one example, where a grid (one dimension for each parameter) is defined and the objective is evaluated for every point in the grid) or depend on values of the objective evaluated for some parameters. For example, for an actual parameter, the neighboring ones are tested, the neighboring parameter with the largest / smallest objective as the actual parameter defined, which is iterated. A more refined processing would e.g. be Gauss-Newton processing. Grid search defines for each parameter a range and a step size (between grid points). The best parameters may be determined by evaluating all grid points and selecting those that maximized or minimized the objective.

For the segmentation, generally different methods or known algorithms may be applied and the processing unit may be configured to segment the one or more anatomical sub-ROIs by use of one or more of a model-based segmentation method, a trained computer system, a trained algorithm, a trained neural network and a classifier (e.g. a classifier with trained parameters, e.g. using thresholds).

The input may be configured to obtain user input of a user interactively marking the anatomical sub-ROIs in at least one of the two or more input images. For instance, as there is no accurate segmentation required, a user may define an ROI by use of a pen or painting (brush) tool, e.g. a coloring or placing a frame around an area. The processing unit may then e.g. be configured to use contours or areas interactively marked by the user as segmented anatomical sub-ROIs or as starting contours or areas for segmenting the one or more anatomical sub-ROIs in the at least one of the two or more input images. This provides a simple but efficient way of segmentation and obtaining sub-ROIs.

In a practical implementation the processing unit may be configured to

- determine at least one characteristic image value, in particular one or more of a mean gray value, a median gray value, a higher order moment, and a standard deviation, for the segmented two or more anatomical sub-ROIs in two or more different composition images representing different weighted compositions of the two or more input images; and
- determine, as quality measure, a contrast-to noise ratio, CNR, for different pairs of anatomical sub-ROIs for the one or more synthesized medical images, in the two or more different composition images based on the determined at least one characteristic image value.

The processing unit may further be configured to synthesize the medical image as a linear or non-linear weighted composition of the two or more input images using the determined set of weights. Hereby, in an embodiment, the processing unit may be configured to clip an input image by setting image values of an input image below or above a respective threshold value for the input image to the respective threshold value and by adding to the clipped input image an additional parameter. The additional parameter may be derived from physics and/or prior knowledge that the image contains some specific information below or above a respective threshold. It might also be optimized to get the best combined image.

In another practical implementation the processing unit is configured to determine, as quality measure, a contrast-to-noise ratio, CNR, and to determine the sum $V_{CNR}$ of the CNRs using a contrast measure based on a power $p \geq 1$ as

$$V_{CNR} = \sum_{(*,\#)} w_{*,\#} \frac{|m_*(\vec{a}) - m_{\#}(\vec{a})|^p}{\sigma_*^p(\vec{a}) + \sigma_{\#}^p(\vec{a})}$$

with $w_{*,\#}$ representing CNR weights, $(*,\#)$ representing the pairs of anatomical sub-ROIs,
$m_*$ representing a mean gray value, $\sigma_*$ representing the standard deviation and $\vec{a}$ representing a weight vector of weights (which may be a linear or non-linear weight vector). Hereby, $w_{*,\#}$ are parameters to make the summed $V_{CNR}$ more flexible. Basically, $w_{*,\#} = 1$ may be set when the ROIs * and # are directly next to each other or $w_{*,\#} = 0$ when the sub-ROIs * and # are distant from each other. It may also be possible to set the weight $w_{*,\#} > 1$ between two sub-ROIs to increase the CNR between them (compared to other regions). It shall be noted here that the weights $w_{*,\#}$ discussed here are not the weights used for synthesizing a medical image.

Further, in this implementation, the processing unit may be configured to determine the weights by maximizing $V_{CNR}$ with respect to the weight vector $\vec{a}$.

In another implementation, the processing unit may be configured to set the sum of the weights to 1 and or to set the CNR weights $w_{*,\#}$ to 1 or use them to interactively optimize the relative contrast. This allows to control relative contrast of the resulting synthesized image. It refers to the weights and is purely a technical means to avoid optimization problems when combinations of weights (parameters) produce an equivalent synthesized image. Images scaled by a scalar can be considered equivalent, because some scaling or look-up-table may be applied when displaying them on the screen. CNR is not changed by scaling.

In an embodiment, the input image data set may be a spectral image data set, in particular a spectral CT image data set or a photon counting CT image data set, which may depend on the kind of detector that has been used for acquiring the spectral image data set. Further, the spectral image data set may comprise two or more of a spectral base image, a monoenergetic image (e.g. monoE40KeV, monoE60KeV, etc.) image, an iodine map image, and a Zeff image.

In other embodiment, the input image data set may be a multi-contrast image data set, in particular a multi-contrast MR image data set.

The proposed imaging system comprises, besides the device described above, an imaging unit, e.g. a spectral CT scanner or a multi-contrast MR scanner, and a display unit, such as a screen of a computer or workstation. In addition, a user interface, e.g. a touch screen, pointer, computer mouse, etc., may be provided that is configured to allow a user to provide user input to interactively mark the anatomical sub-ROIs in at least one of two or more spectral images of the spectral image data set.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of an embodiment of a system according to the present invention.
Fig. 2 shows a schematic diagram of an embodiment of a device according to the present invention.
Fig. 3 shows a schematic diagram of an embodiment of a method according to the present invention.
Fig. 4A shows a conventional CT image.

Fig. 4B shows a monoE40KeV image.

Fig. 4C shows a density map.

Fig. 4D shows a Zeff image.

Fig. 4E shows a iodine map.

Fig. 4F shows a balanced synthesized medical image.

DETAILED DESCRIPTION OF EMBODIMENTS

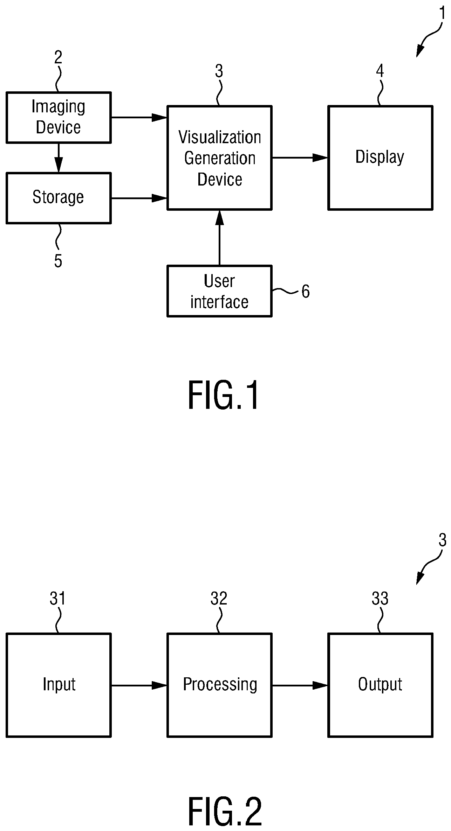

Fig. 1 shows a schematic diagram of an embodiment of a system 1 for synthesizing a medical image of a region of interest (ROI) of a subject according to the present invention. The system 1 comprises an imaging device 2 for acquiring an input image data set of the ROI of the subject, a device 3 for synthesizing a medical image of the ROI of the subject based on the acquired input image data set, and a display unit 4 for displaying the synthesized medical image.

The imaging device 2 may be a CT device, in particular a spectral CT or photon-counting CT device, e.g. a conventionally used CT device for acquiring image data of a subject, e.g. of a certain body region of a patient. The input image data set will then be a spectral image data set, which may be understood as spectral CT image data set or photon-counting CT image data set. The input images will be spectral images, , which may be understood as spectral CT images or photon-counting CT images. In the following explanations reference will be made to this embodiment.

In another embodiment the imaging device 2 may be an MR device that acquires multi-contrast MR images according to other MR protocols generating images with various contrasts. In this embodiment the input image data set will be a multi-contrast MR image data set, and the input images will be multi-contrast MR images or image with different contrast.

Referring to the embodiment of a CT device, the imaging device acquires a spectral image data set comprising two or more spectral images. The spectral image data set may comprise one or more base images, a monoE40KeV image (or other monoenergetic image), an iodine map, a Zeff image, etc., which may be acquired simultaneously or subsequently (almost simultaneously), depending on the implementation of the imaging device, in particular its detector and its radiation source.

The device 3 may e.g. be implemented as or in a processor or computer or on a server or in the cloud, in software and/or hardware. For instance, a programmed processor may be included in the device 3, which may execute a computer program that may be stored in a memory that is accessed by the processor.

The display unit 4 may e.g. be a monitor or screen of a PC, workstation, laptop tablet, etc., e.g. of a computer that represents the device 3 and is carrying out the method for synthesizing a medical image according to the present invention.

The device 3 may directly obtain (i.e. retrieve or receive) the image data set directly from the imaging device 2 or from a storage 5, such as a buffer or memory or image repository, e.g. a patient record stored in a hospital's document management system, e.g. via a wired or wireless network.

A user interface 6, e.g. a touch screen, pointer, computer mouse, etc., may be provided so that a user can provide user input, for instance to interactively mark or correct the contour of the anatomical sub-ROIs in at least one of two or more spectral images of the spectral image data set.

Fig. 2 shows a schematic diagram of an embodiment of a device 3 for synthesizing a medical image of the ROI of the subject according to the present invention. The device may comprise circuitry, e.g. a processor, processing circuitry, a computer, dedicated hardware, etc. that carries out the functions of the device. In another embodiment, as shown in Fig. 2, separate units or elements may be used that together represent circuitry of the device 3.

In this embodiment the device 3 comprises an input unit 31 configured to obtain (received or retrieve) a spectral image data set of the ROI of the subject, the spectral image data set comprising two or more spectral images. The spectral image data set may be obtained, e.g. from the imaging device 2 or the storage 5. For this purpose the input unit 31 may be directly or indirectly (e.g. via a network or bus) coupled or connected to the imaging device 2 or the storage 5. The input unit may thus e.g. be a (wired or wireless) communication interface or data interface, such as a Bluetooth interface, Wi-Fi interface, LAN interface, HDMI interface, direct cable connection, or any other suitable interface allowing data transfer to the device 2.

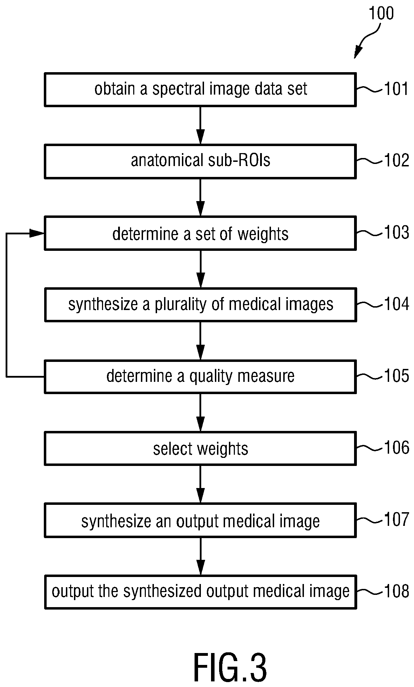

The device 3 further comprises a processing unit 32 configured to carry out the steps of the method according to the present invention as explained below with reference to Fig. 3. The processing unit 32 may be any kind of means configured to process the obtained spectral image data set and to generate a medical image of the ROI of the subject indicating contrast-enhanced regions there from. It may be implemented in software and/or hardware, e.g. as a programmed processor, computer, laptop, PC, workstation, etc.

The device 3 further comprises an output 33 configured to output synthesized output medical image of the ROI generated from the spectral image data set. The output 33 may generally be any interface that provides the generated visualization, e.g. transmits it to another device or provides it for retrieval by another device, e.g. transmits it to another computer or transfers it directly to the display 4 for displaying it. It may thus generally be any (wired or wireless) communication or data interface.

Fig. 3 shows a flow chart of a first embodiment of a method 100 for synthesizing a medical image of the ROI of the subject according to the present invention. The steps of the method may be carried out by the device 3, wherein the main steps of the method are carried out by the processing unit 32. The method may e.g. be implemented as computer program running on a computer or processor.

Main steps of an exemplary embodiment of the present invention are a segmentation of two or more regions (herein called anatomical sub-ROIs) in a spectral image (e.g. a spectral CT image), estimation of mean gray value and noise for the segmented image regions, and determination of the optimal combination of spectral images for viewing. The different steps of the present invention will be explained in more detail below using the example of viewing late-contrast-enhanced cardiac CTA images with left ventricle (LV), left ventricular myocardium (LVM) and scar tissue (ST) as anatomical sub-ROIs.

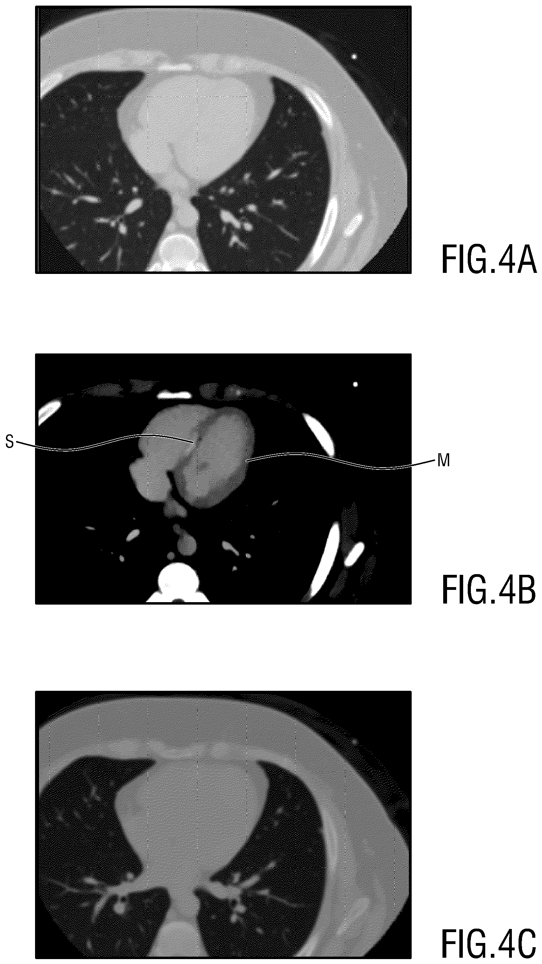

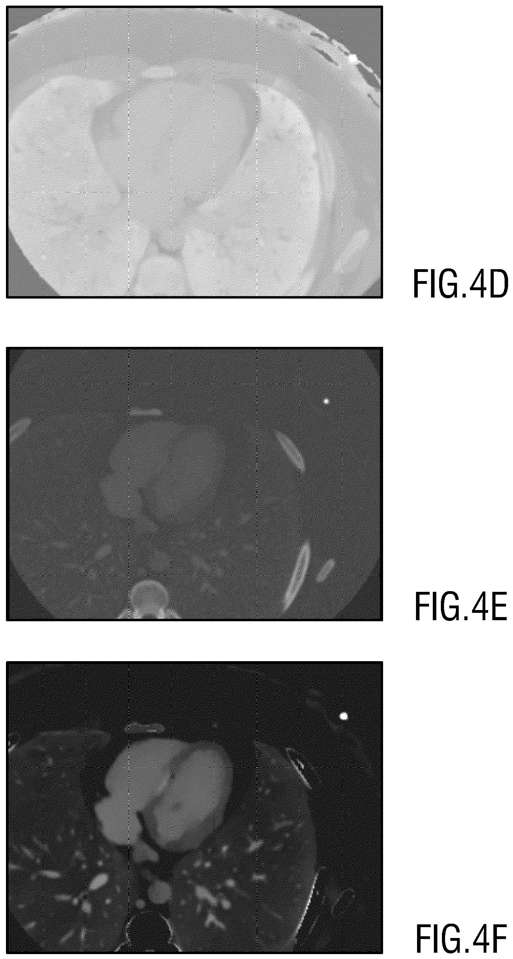

In a first step 101, a spectral image data set of the ROI of the subject is obtained, the spectral image data set comprising two or more spectral images. The spectral image data set may be directly obtained from the imaging device, e.g. a spectral or photon-counting CT scanner, or from a storage, e.g. an image repository. As exemplary images, Figs. 4A-F show different CT images, namely a conventional CT image (Fig. 4A), a monoE40KeV image (Fig. 4B), a density map (Fig. 4C), a Zeff image (Fig. 4D), an iodine map (Fig. 4E), and a balanced synthesized medical image (Fig. 4F). As can be seen by a comparison of Figs. 4A and 4B, blood pool myocardium M and scar tissue S can be much better recognized in the monoE40KeV image of a late contrast enhancement (LCE) spectral CTA scan shown in Fig. 4B.

In a second step 102, one or more anatomical sub-ROIs are segmented in at least one of the two or more spectral images. Segmentation of one sub-ROI may be sufficient if the area inside the segmented sub-ROI and the area outside of the segmented sub-ROI are used as two separate sub-ROIs. As the aim of segmentation is to define the gray value characteristics of the desired structures (sub-ROIs), there is no need for an accurate segmentation, but it is more important to define sub-ROIs with the typical gray value characteristics.

In embodiments, segmentation of the image into different sub-ROIs such LV, LVM and ST can be done automatically using methods such as model-based heart segmentation for LV and LVM and U-nets for ST or U-nets for all sub-ROIs. Either a single spectral CT image (e.g., corresponding to a conventional CT image or a monoenergetic image) or a collection of spectral CT images (e.g., all SBIs) may be used in this step.

Alternatively, the desired sub-ROIs can be marked interactively (e.g., using a painting or contouring tool by the user). In this case, use of a single image (e.g., corresponding to a conventional CT image or a monoenergetic image) for contouring of a specific sub-ROI is preferred, but different images may be used for different sub-ROIs.

In a third step 103, a set (or sequence) of weights (sometimes also referred to as "parameters") for synthesizing medical images from the two or more spectral images using the set of weights is determined. In an exemplary embodiment a grid search may be applied or a predefined set of weights may be used. More refined optimization algorithms (e.g. Gauss Newton) may be used to generate a sequence of weights in dependence of previous results.

In a fourth step 104, one or more (preferably a plurality of) medical images are synthesized from the two or more spectral images using different weights of the determined set of weights. In an embodiment, a weighted composition of the two or more spectral images using different weights may be made. In other embodiments, a combination of images may be done e.g. as $I(x) = w(x) * I1(x) + (1-w(x)) * I2(x); w(x) = \{0$ for $I2(x) \geq a; 1$ for $I2(x) \leq 0; (a - I2(x)) / a$ else$\}$.

In a fifth step 105, a quality measure for different pairs of anatomical sub-ROIs for the one or more synthesized medical images is determined, the quality measure indicating differences between the anatomical sub-ROIs of a pair. In a non-limiting embodiment, the contrast-to noise ratio, CNR, for different pairs of anatomical sub-ROIs is determined as quality measure for a number of images synthesized with different weights. In another embodiment a scalar quantity characterizing the gray value differences between two sub-ROIs is determined as quality measure. When images are synthesized linearly, the CNR may be computed by combining characteristic image values. When images are synthesized non-linearly, it may be necessary to compute the synthesized image in the ROIs to determine the characteristic image value.

In a sixth step 106, weights are select from the determined set of weights for which the sum of the quality measures determined for the different pairs of anatomical sub-ROIs is maximal.

Steps 103 to 105 may be iterated two or more times, as indicated by the arrow from step 105 to step 103 to carry out the optimization. For instance, such an optimization iteration may by seen like going uphill/downhill on a grid. Using a set of parameters images are synthesized for neighboring parameter sets on a grid. Then, the maximum/minimum are selected and the steps are iterated. The iterations are stopped when the quality measure, e.g. CNR, for neighboring parameters on the grid is smaller than at the current grid point. In each iteration, a single medical image may be synthesized in the fourth step 104.

In a seventh step 107, an output medical image is synthesized from the two or more spectral images using the selected weights. The synthesis may be carried out in the same manner as in the fourth step 104. As an alternative, one of the one or more synthesized medical images may be selected as output medical image based on the selected weights, i.e., either the synthesized image may be selected that has been synthesized using the weights that have been selected in step 106. In another embodiment, only one medical image may be synthesized, and the best image - with respect to the quality measure, e.g. with respect to CNR - of the input images (used for synthesis) and the synthesized medical image may be selected as output medical image.

In an eighth step 108, the synthesized output medical image is outputted, e.g. provided to a monitor for display or transmitted to a computer for further processing, storage or display.

It shall be noted that steps 103 and 104 may be carried out by an external device, e.g. the imaging device 2 shown in Fig. 2. In this case, both the device 2 and the device 3 each has a processing sub-unit which together form the processing unit that carries out the steps of the disclosed method. For instance, the processing sub-unit of the imaging device 2 may use/determine (step 103) a predetermined set of weights, e.g. take them from a database or a look-up table to synthesize (step 104) the one or more synthesized images. The one or more synthesized images and, optionally, the used set of weights may then be provided to the processing unit 32 of the device 3 as further input.

In an exemplary implementation, steps 102 to 106 may be carried out as follows in order to estimate the mean gray value and noise for the sub-ROIs. The segmentation result (step 102) is a (compact) set or region of voxels $L_{LV}$, $L_{LVM}$ and $L_{ST}$ defining sub-ROIs of the LV, LVM and ST. In addition, it may be assumed that some spectral image

$$I(\vec{x}; \vec{a}) = a_1 I_1(\vec{x}) + \cdots + a_n I_n(\vec{x})$$

can be composed from SBIs $I_1, \ldots, I_n$ given the linear weight parameters $\vec{a}$, which may be predetermined or determined in step 103. Alternatively, non-linear superpositions

$$I(\vec{x}; \vec{a}) = \psi(I_1(\vec{x}), \ldots, I_n(\vec{x}); \vec{a})$$

are possible, for instance by clipping an SBI (i.e., setting gray values below or above a threshold for the SBI to the threshold) and adding the clipped image with an additional parameter. In addition, it can be envisaged that $I(\vec{x}; \vec{a})$ can be composed in further non-linear ways out of the base images $I_1, \ldots, I_n$ e.g., by a generic multi-channel neural network $\psi$ and that the number of parameters $a_i$ is larger than the number of base images. This kind of processing can be used to implement steps 104 and 107.

In step 105, gray value characteristics such as the mean gray value $m_*$ or the standard deviation $\sigma_*$ within a particular region $L_*$ can be defined for each spectral image $I(\vec{x}; \vec{a})$ according to:

$$m_*(\vec{a}) = \mathrm{mean}\{I(\vec{x}; \vec{a}) | \vec{x} \in L_*\}$$

$$\sigma_*(\vec{a}) = \mathrm{std}\{I(\vec{x}; \vec{a}) | \vec{x} \in L_*\}$$

Numerous variants to compute statistical gray value properties can be envisaged as well. Instead of the mean gray value, the median gray value, higher order moments or other image features $\phi$ may be used. The standard deviation might be computed directly from gray values or by fitting a (Gaussian) distribution to a histogram.

The determination of the optimal combination of spectral CT images for viewing may be done as follows. A measure for the CNR between the three tissues can, for instance, be defined by summing over pairs (∗, #) of tissues using a contrast measure based on a power $p \in \{1,2,3, .. \}$

$$V_{CNR} = \sum_{(*,\#) \in \{(LV,LVM),(LV,LCE),(LCE,LVM)\}} w_{*,\#} \frac{|m_*(\vec{a}) - m_\#(\vec{a})|^p}{\sigma_*^p(\vec{a}) + \sigma_\#^p(\vec{a})}$$

and an optimal or improved CNR can be determined by maximizing $V_{CNR}$ with respect to the parameters $\vec{a}$, which represents step 106. Further, boundary conditions such as $a_1 + \cdots + a_N = 1$ may be required to limit the scale of the optimized image. The weights $w_{*,\#}$ may be set to 1 or used to interactively optimize the relative contrast. This is indicated by the arrow from step 105 to step 103 in Fig. 3, which represents the optimization iteration.

The image with optimized parameters $\vec{a}$ is afterwards outputted (step 108) and may be displayed for diagnosis.

Further discriminant objective functions can be used in step 105, e.g., linear discriminant analysis (LDA), support vector machine (SVM) accuracy, logistic regression, etc.

The present invention may be applied to enable or facilitate a diagnosis in different medical applications and for different organs, such as myocardial diseases, heart diseases, liver perfusion analysis and brain stroke analysis. Other anatomical sub-ROIs and other numbers of anatomical sub-ROIs than used in the exemplary embodiment described above may be used.

The image data set may preferably be a CT image data set where soft tissue contrast is low (compared to MR), obtained by a spectral CT or photon counting CT. In other embodiments a multi-contrast MR image data set or image data set obtained by other MR protocols generating images with various contrasts may be used.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for synthesizing a medical image of a region of interest, ROI, of a subject, the device comprising:

   - an input (31) configured to obtain an input image data set of the ROI of the subject, the spectral image data set comprising two or more input images;
   - a processing unit (32) configured to

     - segment one or more anatomical sub-ROIs in at least one of the two or more input images;
     - etermine a set of weights for synthesizing medical images from the two or more input images using the set of weights;
     - synthesize one or more medical images from the two or more input images using different weights of the determined set of weights;
     - determine a quality measure for different pairs of anatomical sub-ROIs for the one or more synthesized medical images, the quality measure indicating differences between the anatomical sub-ROIs of a pair;
     - select weights from the determined set of weights for which the sum of the quality measures determined for the different pairs of anatomical sub-ROIs is maximal; and
     - synthesize an output medical image from the two or more input images using the selected weights or select one of the one or more synthesized medical images as output medical image based on the selected weights; and

   - an output (33) configured to output the synthesized output medical image.

2. Device as claimed in claim 1,
   wherein the processing unit (32) is configured to determine, as quality measure, a contrast-to-noise ratio, CNR, or a scalar quantity characterizing the gray value differences between two anatomical sub-ROIs of a pair of anatomical sub-ROIs.

3. Device as claimed in any one of the preceding claims,
   wherein the processing unit (32) is configured to synthesize the one or more medical images as weighted compositions of the two or more input images using different weights of the determined set of weights and/or to synthesize the output medical image as weighted composition from the two or more input images using the selected weights.

4. Device as claimed in any one of the preceding claims,
   wherein the processing unit (32) is configured to determine the set of weights by use of a grid search or from a predetermined set of weights or by use of an optimization of a previously used set of weights.

**5.** Device according to any one of the preceding claims,
wherein the processing unit (32) is configured to segment the one or more anatomical ROIs by use of one or more of a model-based segmentation method, a trained computer system, and a trained algorithm, a trained neural network and a classifier.

**6.** Device according to any one of the preceding claims,
wherein the processing unit (32) is configured to

- determine at least one characteristic image value, in particular one or more of a mean gray value, a median gray value, a higher order moment, and a standard deviation, for the segmented two or more anatomical sub-ROIs in two or more different composition images representing different weighted compositions of the two or more input images; and
- determine, as quality measure, a contrast-to noise ratio, CNR, for different pairs of anatomical sub-ROIs for the one or more synthesized medical images, in the two or more different composition images based on the determined at least one characteristic image value.

**7.** Device according to any one of the preceding claims,
wherein the processing unit (32) is configured to synthesize the medical image as a linear or non-linear weighted composition of the two or more input images using the determined set of weights.

**8.** Device according to claim 7,
wherein the processing unit (32) is configured to clip an input image by setting image values of an input image below or above a respective threshold value for the input image to the respective threshold value and adding to the clipped input image an additional parameter.

**9.** Device according to any one of the preceding claims,
wherein the processing unit (32) is configured to determine, as quality measure, a contrast-to-noise ratio, CNR, and to determine the sum $V_{CNR}$ of the CNRs using a contrast measure based on a power $p \geq 1$ as

$$V_{CNR} = \sum_{(*,\#)} w_{*,\#} \ \frac{|m_*(\vec{a})-m_\#(\vec{a})|^p}{\sigma_*^p(\vec{a})+\sigma_\#^p(\vec{a})}$$

with $w_{*,\#}$ representing CNR weights, $(*,\#)$ representing the pairs of anatomical sub-ROIs, $m_*$ representing a mean gray value, $\sigma_*$ representing the standard deviation and $\vec{a}$ representing a weight vector of weights.

**10.** Device according to claim 9,
wherein the processing unit (32) is configured to determine the weights by maximizing $V_{CNR}$ with respect to the linear weight vector $\vec{a}$ and/or to set the sum of the weights to 1 and or to set the CNR weights w_(*,#) to 1 or use them to interactively optimize the relative contrast.

**11.** Device according to claim 9 or 10,
wherein the input image data set is a spectral image data set, in particular a spectral CT image data set, or a multi-contrast image data set, in particular a multi-contrast MR image data set.

**12.** Imaging system comprising:

- an imaging device (2) configured to acquire an input image data set of a region of interest, ROI, of a subject, the input image data set comprising two or more input images;
- a device (3) according to any one of the preceding claims for synthesizing a medical image of the ROI of the subject based on the acquired input image data set; and
- a display unit (4) configured to display the synthesized medical image.

**13.** Imaging system according to claim 12,
further comprising a user interface (6) configured to allow a user to provide user input to interactively mark the anatomical sub-ROIs in at least one of two or more input images of the input image data set.

14. Method for synthesizing a medical image of a region of interest, ROI, of a subject, the method comprising:

- obtaining an input image data set of the ROI of the subject, the input image data set comprising two or more input images;
- segmenting one or more anatomical sub-ROIs in at least one of the two or more input images;
- determining a set of weights for synthesizing medical images from the two or more input images using the set of weights;
- synthesizing one or more medical images from the two or more input images using different weights of the determined set of weights;
- determining a quality measure for different pairs of anatomical sub-ROIs for the one or more synthesized medical images, the quality measure indicating differences between the anatomical sub-ROIs of a pair;
- selecting weights from the determined set of weights for which the sum of the quality measures determined for the different pairs of anatomical sub-ROIs is maximal;
- synthesizing an output medical image from the two or more input images using the selected weights or selecting one of the one or more synthesized medical images as output medical image based on the selected weights; and
- outputting the synthesized output medical image.

15. Computer program comprising program code means for causing a computer to carry out the steps of the method as claimed in claim 14 when said computer program is carried out on the computer.

FIG.1

FIG.2

EP 4 459 543 A1

| obtain a spectral image data set | ~101 |
| anatomical sub-ROIs | ~102 |
| determine a set of weights | ~103 |
| synthesize a plurality of medical images | ~104 |
| determine a quality measure | ~105 |
| select weights | ~106 |
| synthesize an output medical image | ~107 |
| output the synthesized output medical image | ~108 |

FIG.3

FIG.4A

FIG.4B

FIG.4C

FIG.4D

FIG.4E

FIG.4F

Europäisches
Patentamt

European
Patent Office

Office européen
des brevets

## EUROPEAN SEARCH REPORT

Application Number

**EP 23 17 0911**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/110583 A1 (LEE CHANG-LAE [KR] ET AL) 15 April 2021 (2021-04-15) * the whole document * ----- | 1-15 | INV. G06T5/50 A61B6/00 H04N23/80 |
| A | US 2021/267563 A1 (SATTARIVAND MIKE [CA] ET AL) 2 September 2021 (2021-09-02) * the whole document * ----- | 1-15 | |
| A | US 2021/153835 A1 (KARIM KARIM S [CA] ET AL) 27 May 2021 (2021-05-27) * the whole document * ----- | 1-15 | |

TECHNICAL FIELDS
SEARCHED      (IPC)

G06T
A61B
H04N

The present search report has been drawn up for all claims

| Place of search | Date of completion of the search | Examiner |
|---|---|---|
| Munich | 10 October 2023 | Mohedano del Pozo, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 17 0911

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2023

| Patent document cited in search report | Publication date | Patent family member(s) | Publication date |
|---|---|---|---|
| US 2021110583 A1 | 15-04-2021 | EP 3644282 A1 | 29-04-2020 |
| | | KR 20190015084 A | 13-02-2019 |
| | | US 2021110583 A1 | 15-04-2021 |
| US 2021267563 A1 | 02-09-2021 | EP 3806747 A1 | 21-04-2021 |
| | | US 2021267563 A1 | 02-09-2021 |
| | | WO 2019237179 A1 | 19-12-2019 |
| US 2021153835 A1 | 27-05-2021 | AU 2020390867 A1 | 09-06-2022 |
| | | BR 112022010084 A2 | 30-08-2022 |
| | | CA 3159717 A1 | 03-06-2021 |
| | | CN 114930197 A | 19-08-2022 |
| | | EP 4066022 A1 | 05-10-2022 |
| | | JP 2023503326 A | 27-01-2023 |
| | | KR 20220104774 A | 26-07-2022 |
| | | US 2021153835 A1 | 27-05-2021 |
| | | WO 2021102576 A1 | 03-06-2021 |

EPO FORM P0459

For more details about this annex : see Official Journal of the European Patent Office, No. 12/82